# EUROPEAN PATENT APPLICATION

(11) **EP 4 803 114 A1**
(43) Date of publication of application: **09.09.2026**
(21) Application number: 26162464.7
(22) Date of filing: 05.03.2026
(51) Int. Cl.: A61M 1/34, A61M 1/02

(54) **METHODS OF UTILIZING A PRESSURE SENSOR TO DETECT ANOMALOUS APHERESIS PROCEDURE EVENTS**

(30) Priority: 06.03.2025 US 202563767813 P
(71) Applicant: Fenwal, Inc., Lake Zurich, IL 60047 (US)
(72) Inventor: MAHER, Jeffrey R., Lake Zurich, 60047 (US); QUEZADA, Francesca S., Lake Zurich, 60047 (US); DICOLA, Nicholas R., Lake Zurich, 60047 (US)
(74) Representative: Maikowski & Ninnemann Patentanwälte Partnerschaft mbB

(57) **Abstract**

A fluid processing procedure, such as an apheresis procedure, is performed by a fluid processing device including one or more components configured to direct fluid flow, a container pressure sensor system, and a controller operably connected to the container pressure sensor system. A method of determining a flow condition in the fluid processing procedure includes receiving a signal from the container pressure sensor system indicative of a pressure in a tube fluidically connected to and directly below a fluid container, determining a flow condition using the received signal, and controlling the at least one of the one or more components based on the determined flow condition.

## Description

### Field of the Disclosure

The present disclosure relates to fluid processing systems and methods. More particularly, the present disclosure relates to fluid processing methods using a pressure sensor positioned on and/or associated with a flow path to determine a flow condition in the system.

### Description of Related Art

Various blood processing systems now make it possible to collect particular blood constituents, rather than whole blood, from a blood source. Typically, in such systems, whole blood is drawn from a blood source, the particular blood component or constituent is separated, removed, and collected, and the remaining blood constituents are returned to the blood source.

Whole blood is typically separated into its constituent components through centrifugation. This requires that the whole blood be passed through a centrifuge after it is withdrawn from, and before it is returned to, the source. To avoid contamination and possible infection of the source, the blood is preferably contained within a sealed, sterile fluid flow circuit during the entire centrifugation process. Typical blood processing systems thus include a permanent, reusable centrifuge assembly containing the hardware (drive system, pumps, valve actuators, programmable controller, and the like) that spins and pumps the blood, and a disposable, sealed and sterile fluid flow circuit that is mounted in cooperation on the hardware. The centrifuge assembly engages and spins a disposable centrifuge chamber of the fluid flow circuit during a collection procedure. The blood, however, makes actual contact only with the fluid flow circuit, which assembly is used only once and then discarded.

As the whole blood is spun by the centrifuge, the heavier (greater specific gravity) components, such as red blood cells, move radially outwardly away from the center of rotation toward the outer or "high-g" wall of the separation chamber. The lighter (lower specific gravity) components, such as plasma, migrate toward the inner or "low-g" wall of the separation chamber. Various ones of these components can be selectively removed from the whole blood by forming appropriately located channeling seals and outlet ports in the separation chamber.

A known blood processing procedure includes several stages in which the centrifuge and the various pumps, valves and clamps may be operated to direct a fluid through different flow paths depending on the stage. In the various stages, fluid may also be drawn from or delivered to a fluid container. The fluid processing device may end one stage and begin another based on an amount of fluid in a container, for example, if the container is empty or full. Such a determination is commonly made according to a detected weight of the container or containers, according to a weight sensor.

### Summary

There are several aspects of the present subject matter which may be embodied separately or together in the devices and methods described and claimed below. These aspects may be employed alone or in combination with other aspects of the subject matter described herein, and the description of these aspects together is not intended to preclude the use of these aspects separately or the claiming of such aspects separately as set forth in the claims appended hereto.

In one aspect, a fluid processing device includes one or more components configured for controlling flow through a flow circuit, the one or more components including one or more of a centrifuge, a pump system and a valve system. The device also includes a container pressure sensor system including one or more pressure sensors configured to detect the pressure exerted by a container in a flow path associated with the fluid container The container pressure sensor system is configured to detect and transmit signals indicative of a pressure within the tube or flow path, wherein the pressure corresponds to a pressure exerted by a volume of fluid in the associated container. The fluid processing device further includes a controller operably connected to the container pressure sensor system and configured to receive the signals transmitted from the container pressure sensor system, determine a flow condition using the received signals, and control the one or more components based on the determined flow condition.

In another aspect, a fluid processing system includes a fluid processing device and a disposable fluid flow circuit configured for selective arrangement on the fluid processing device for the fluid processing device to perform a procedure. The fluid processing device includes one or more components including a pump system, a valve system, and a centrifuge, a container pressure sensor system including one or more container pressure sensors, and a controller operably connected to the container pressure sensor system. The disposable fluid flow circuit includes one or more fluid containers and one or more flow paths fluidically connected to the one or more fluid containers to allow fluid flow into and out of the one or more containers. The disposable fluid flow circuit is associated with the fluid processing device such that a container pressure sensor of the container pressure sensor system is arranged at a flow path associated with the one or more fluid containers to which to the tube is fluidically connected. The container pressure sensor system is configured to detect and transmit signals indicative of a pressure within the tube, wherein the pressure corresponds to a pressure exerted by a volume of fluid in the corresponding fluid container. The controller is configured to receive the signals transmitted from the container pressure sensor system, determine a flow condition using the received signals, and control the one or more components based on the determined flow condition.

In yet another aspect, a method of determining a flow condition in a fluid processing procedure is performed by a fluid processing device. The fluid processing device includes one or more components configured to direct fluid flow, a container pressure sensor system comprising one or more pressure sensors, and a controller operably connected to the container pressure sensor system and at least one of the one or more components. The method includes receiving a signal from a container pressure sensor of the container pressure sensor system indicative of a pressure in a flow path fluidically connected to and directly below a fluid container, determining a flow condition using the received signal, and controlling the at least one component of the one or more components based on the determined flow condition.

### Brief Description of the Drawings

FIG. 1 is a perspective view of an exemplary fluid processing device according to an aspect of the present disclosure.
FIG. 2 is a schematic view of an exemplary disposable fluid flow circuit that may be mounted to the fluid processing device of FIG. 1 to complete a fluid processing system according to an aspect of the present disclosure.
FIG. 3 is a block diagram showing an arrangement of container pressure sensors connected to a controller of the exemplary fluid processing device of FIG. 1.
FIG. 4 is a block diagram showing an example of a method for determining a flow condition using a pressure sensor.
FIG. 5 shows one example of a cassette for use and mounting onto peristaltic pumps of the system disclosed herein.
FIG. 6 shows an example of an alternative cassette for use with pneumatic pumps (and optionally additional peristaltic pumps) of the system disclosed herein.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

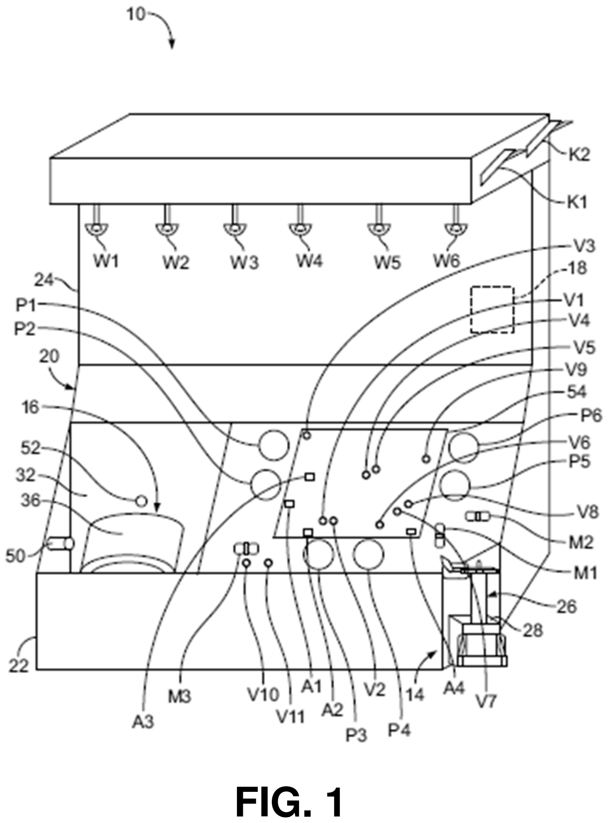

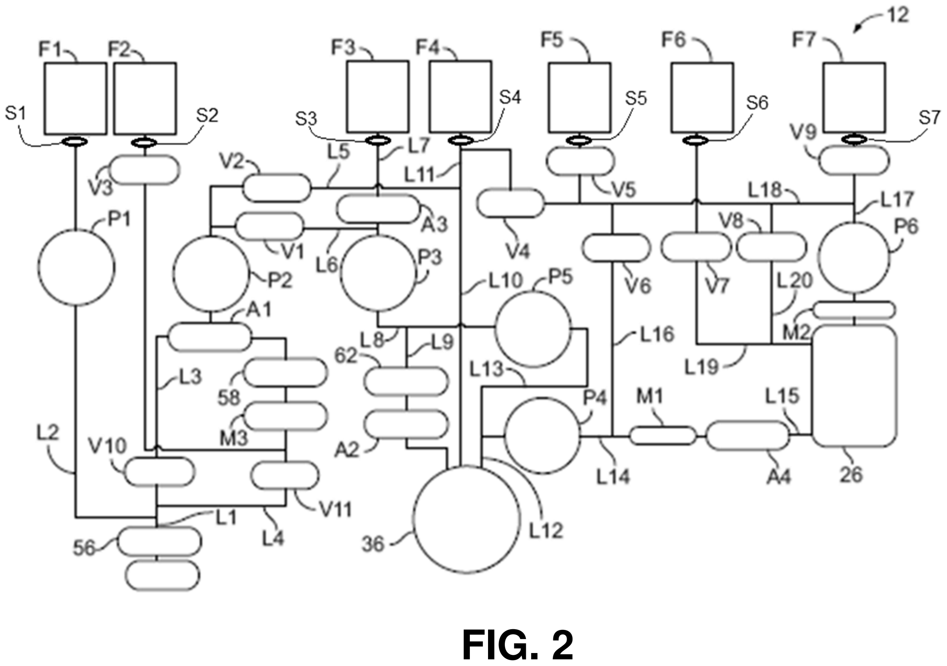

FIGS. 1 and 2 show components of a biological fluid processing system that embodies various aspects of the present subject matter. Use of the system for separating blood or other biological fluid into two or more components and collecting at least one of the components will be described herein, though it should be understood that systems according to the present disclosure can be used for processing a variety of different biological fluids.

Generally speaking, the system includes two principal components, a durable and reusable biological fluid processing device 10 (FIG. 1) and a disposable fluid flow circuit 12 (FIG. 2). The illustrated biological fluid processing device 10 includes a spinning membrane separator drive unit 14, a centrifuge or centrifugal separator 16, additional components that control fluid flow through the disposable fluid flow circuit 12, and a controller 18, which governs the operation of the other components of the biological fluid processing device 10 to perform a biological fluid processing procedure. While the principles described herein may be employed when using the biological fluid processing device 10 of FIG. 1, it should be understood that these same principles may be applied to other biological fluid processing devices, including devices employing single separation technologies or approaches.

### I. The Durable Biological Fluid Processing Device

The biological fluid processing device 10 (FIG. 1) is configured as a durable item that is capable of long-term use. It should be understood that the fluid processing device 10 of FIG. 1 is merely exemplary of one possible configuration and that biological fluid processing devices according to the present disclosure may be differently configured.

In the illustrated embodiment, the fluid processing device 10 is embodied in a single housing or case 20. The illustrated case 20 includes a generally horizontal portion 22 (which may include an inclined or angled face or upper surface for enhanced visibility and ergonomics) and a generally vertical portion 24. The spinning membrane separator drive unit 14 and the centrifugal separator 16 are shown as being incorporated into the generally horizontal portion 22 of the case 20, while the controller 18 is shown as being incorporated into the generally vertical portion 24.

### A. Spinning Membrane Separator Drive Unit

The fluid processing device 10 includes a spinner support or spinning membrane separator drive unit 14 for accommodating a generally cylindrical spinning membrane separator 26 of the fluid flow circuit 12. U.S. Patent No. 5,194,145 (which is hereby incorporated herein by reference) describes an exemplary spinning membrane separator drive unit that would be suitable for incorporation into the fluid processing device 10, but it should be understood that the spinning membrane separator drive unit 14 may be differently configured without departing from the scope of the present disclosure.

The illustrated spinning membrane separator drive unit 14 has a base 28 configured to receive a lower portion of the spinning membrane separator 26 and an upper end cap 30 to receive an upper portion of the spinning membrane separator 26. Preferably, the upper end cap 30 is positioned directly above the base 28 to orient a spinning membrane separator 26 received by the spinning membrane separator drive unit 14 vertically and to define a vertical axis about which the spinning membrane separator 26 is spun. While it may be advantageous for the spinning membrane separator drive unit 14 to vertically orient a spinning membrane separator 26, it is also within the scope of the present disclosure for the spinning membrane separator 26 to be differently oriented when mounted to the fluid processing device 10.

In one embodiment, one of the base 28 and upper end cap 30 of the spinning membrane separator drive unit 14 is movable with respect to the other, which may allow differently sized spinning membrane separators 26 to be received by the spinning membrane separator drive unit 14. For example, the upper end cap 30 may be translated vertically with respect to the base 28 and locked in a plurality of different positions, with each locking position corresponding to a differently sized spinning membrane separator 26.

At least one of the base 28 and the upper end cap 30 is configured to spin one or more components of the spinning membrane separator 26 about the axis defined by the spinning membrane separator drive unit 14. The mechanism by which the spinning membrane separator drive unit 14 spins one or more components of the spinning membrane separator 26 may vary without departing from the scope of the present disclosure. In one embodiment, a component of the spinning membrane separator 26 to be spun includes at least one element configured to be acted upon by a magnet (e.g., a metallic material), while the spinning membrane separator drive unit 14 includes a magnet (e.g., a series of magnetic coils or semi-circular arcs). By modulating the magnetic field acting upon the aforementioned element of the spinning membrane separator 26, the component or components of the spinning membrane separator 26 may be made to spin in different directions and at varying speeds. In other embodiments, different mechanisms may be employed to spin the component or components of the spinning membrane separator 26.

Regardless of the mechanism by which the spinning membrane separator drive unit 14 spins the component or components of the spinning membrane separator 26, the component or components of the spinning membrane separator 26 is/are preferably spun at a speed that is sufficient to create Taylor vortices in a gap between the spinning component and a stationary component of the spinning membrane separator 26 (or a component that spins at a different speed). Fluid to be separated within the spinning membrane separator 26 flows through this gap, and filtration may be dramatically improved by the creation of Taylor vortices.

### B. Centrifugal Separator

As for the centrifugal separator 16, it includes a centrifuge compartment 32 that receives a centrifugal separation chamber 36 of the fluid flow circuit 12, as well as other components of the centrifugal separator 16. Further details as to the centrifugal separator are set forth in PCT Patent Application Publication No. WO 2018/053217 A1, which is hereby incorporated herein by reference.

Fluid (e.g., anticoagulated whole blood) is introduced into the centrifugal separation chamber 36 by an umbilicus, with the fluid being separated into a layer of less dense components (e.g., platelet-rich plasma, in the case of blood being separated) and a layer of more dense components (e.g., packed red blood cells) within the centrifugal separation chamber 36 as a result of centrifugal forces as it rotates. Components of an interface monitoring system may be positioned within the centrifuge compartment 32 to oversee separation of fluid within the centrifugal separation chamber 36. The interface monitoring system may include a light source 50 and a light detector 52, which is positioned and oriented to receive at least a portion of the light emitted by the light source 50.

The orientation of the various components of the interface monitoring system depends at least in part on the particular configuration of the centrifugal separation chamber 36. In general, though, the light source 50 emits a light beam (e.g., a laser light beam) through the separated fluid components within the centrifugal separation chamber 36 (which may be formed of a material that substantially transmits the light or at least a particular wavelength of the light without absorbing it). A portion of the light reaches the light detector 52, which transmits a signal to the controller 18 that is indicative of the location of an interface between the separated fluid components. If the controller 18 determines that the interface is in the wrong location (which can affect the separation efficiency of the centrifugal separator 16 and/or the quality of the separated fluid components), then it can issue commands to the appropriate components of the biological fluid processing device 10 to modify their operation so as to move the interface to the proper location.

### C. Other Components of the Biological Fluid Processing Device

In addition to the spinning membrane separator drive unit 14 and the centrifugal separator 16, the biological fluid processing device 10 may include other components compactly arranged to aid fluid processing.

The generally horizontal portion 22 of the case 20 of the illustrated fluid processing device 10 includes a cassette station 54, which accommodates a flow control cassette of the fluid flow circuit 12. In one embodiment, the cassette station 54 is similarly configured to the cassette station of U.S. Patent No. 5,868,696 (which is hereby incorporated herein by reference) but is adapted to include additional components and functionality. The illustrated cassette station 54 includes a plurality of clamps or valves V1-V9 (which are collectively referred to herein as the "valve system" of the biological fluid processing system 10), which move between a plurality of positions (e.g., between a retracted or lowered position and an actuated or raised position) to selectively contact or otherwise interact with corresponding valve stations of the flow control cassette of the fluid flow circuit 12. Depending on the configuration of the fluid flow circuit 12, its cassette may not include a valve station for each valve V1-V9 of the cassette station 54, in which case fewer than all of the valves V1-V9 will be used in a fluid processing procedure.

In the actuated position, a valve V1-V9 engages the associated valve station to prevent fluid flow through that valve station (e.g., by closing one or more ports associated with the valve station, thereby preventing fluid flow through that port or ports). In the retracted position, a valve V1-V9 is disengaged from the associated valve station (or less forcefully contacts the associated valve station than when in the actuated position) to allow fluid flow through that valve station (e.g., by opening one or more ports associated with the valve station, thereby allowing fluid flow through that port or ports). Additional clamps or valves V10 and V11 of the valve system may be positioned outside of the cassette station 54 to interact with portions of valve stations (which may be lengths of tubing) of the fluid flow circuit 12 to selectively allow and prevent fluid flow therethrough. The valves V1-V9 and corresponding valve stations of the cassette station 54 and cassette may be differently configured and operate differently from the valves V10 and V11 and the valve stations that are spaced away from the cassette station 54.

The cassette station 54 may be provided with additional components, such as pressure sensors A1-A4, which interact with sensor stations of the cassette to monitor the pressure at various locations of the fluid flow circuit 12. For example, if the fluid source is a human donor, one or more of the pressure sensors A1-A4 may be configured to monitor the pressure of the donor's vein during blood draw and return. Other pressure sensors A1-A4 may monitor the pressure of the spinning membrane separator 26 and the centrifugal separation chamber 36. The controller 18 may receive signals from the pressure sensors A1-A4 that are indicative of the pressure within the fluid flow circuit 12 and, if a signal indicates a low- or high-pressure condition, the controller 18 may initiate an alarm or error condition to alert an operator to the condition and/or to attempt to bring the pressure to an acceptable level without operator intervention.

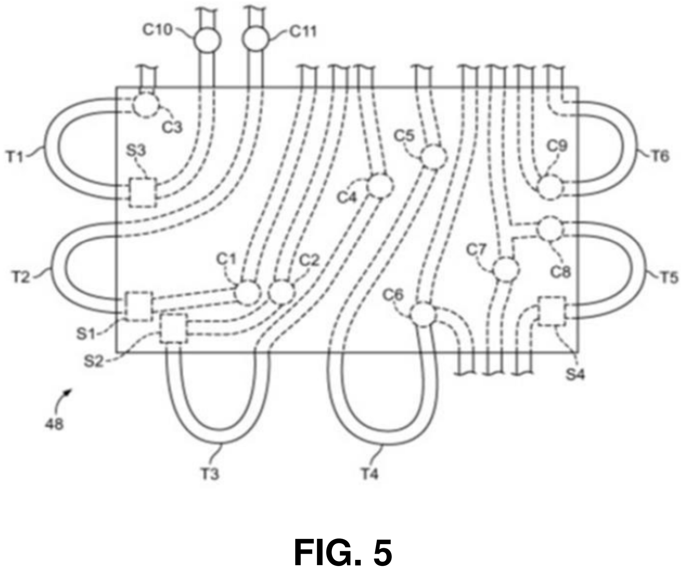

The biological fluid processing device 10 may also include a plurality of pumps P1-P6 (which are collectively referred to herein as the "pump system" of the biological fluid processing device 10) to cause fluid to flow through the fluid flow circuit 12. The pumps P1-P6 may be differently or similarly configured and/or function similarly or differently from each other. In the illustrated embodiment, the pumps P1-P6 are configured as peristaltic pumps, which may be generally configured as described in U.S. Patent No. 5,868,696. Each pump P1-P6 engages a different tubing loop (shown, for example, in Fig. 5) extending from a side surface of the flow control cassette 48 and may be selectively operated under command of the controller 18 to cause fluid to flow through a portion of the fluid flow circuit 12. In one embodiment, all or a portion of the cassette station 54 may be capable of translational motion in and out of the case 20 to allow for automatic loading of the tubing loops into the associated pump P1-P6. In another exemplary embodiment, rather than employing peristaltic pumps, pneumatic pumps may be employed, with actuators incorporated into the cassette station 54 interacting with suitably configured portions of the fluid flow circuit 12 (e.g., pump stations of a cassette mounted to the cassette station 54) to convey fluid through the fluid flow circuit 12. One or more flow sensors (not shown) may be provided with one or more pumps P1-P6 and may be co-located with the pump(s) and/or downstream from pump(s) to measure, for example, a flow rate of the pumped fluid. The flow sensor(s) may be communicably connected to the controller 18.

The illustrated biological fluid processing device 10 also includes a spinner inlet sensor M1 for determining one or more properties of a fluid flowing into a spinning membrane separator 26 mounted within the spinning membrane separator drive unit 14. If the fluid flowing into the spinning membrane separator 26 is whole blood (which may include anticoagulated whole blood), the spinner inlet sensor M1 may be configured to determine the hematocrit of the blood flowing into the spinning membrane separator 26. If the fluid flowing into the spinning membrane separator 26 is platelet-rich plasma, the spinner inlet sensor M1 may be configured to determine the platelet concentration of platelet-rich plasma flowing into the spinning membrane separator 26. The spinner inlet sensor M1 may detect the one or more properties of a fluid by optically monitoring the fluid as it flows through tubing of the fluid flow circuit 12, or by any other suitable approach. The controller 18 may receive signals from the spinner inlet sensor M1 that are indicative of the one or more properties of fluid flowing into the spinning membrane separator 26 and use the signals to optimize the fluid processing procedure based upon that property or properties. If the property or properties is/are outside of an acceptable range, then the controller 18 may initiate an alarm or error condition to alert an operator to the condition. A suitable device and method for monitoring hematocrit and/or platelet concentration is described in U.S. Patent No. 6,419,822 (which is hereby incorporated herein by reference), but it should be understood that a different approach may also be employed for monitoring one or more properties of a fluid or fluid component flowing into the spinning membrane separator 26.

The illustrated biological fluid processing device 10 further includes a spinner outlet sensor M2, which accommodates tubing of the fluid flow circuit 12 that flows a separated fluid component out of the spinning membrane separator 26. The spinner outlet sensor M2 monitors the separated fluid component to determine one or more properties thereof, and may do so by optically monitoring the separated fluid component as it flows through the tubing or by any other suitable approach. In one embodiment, separated plasma flows through the tubing, in which case the spinner outlet sensor M2 may be configured to determine the amount of cellular blood components in the plasma and/or whether the plasma is hemolytic and/or lipemic. This may be done using an optical monitor of the type described in U.S. Patent No. 8,556,793 (which is hereby incorporated herein by reference) that measures the optical density of the fluid in the associated tubing, or by any other suitable device and/or method.

The illustrated fluid processing device also includes an air detector M3 (e.g., an ultrasonic bubble detector), which accommodates tubing of the fluid flow circuit 12 that flows fluid to a recipient. It may be advantageous to prevent air from reaching the recipient, whether a human recipient (e.g., the same human that serves as the blood source) or a non-human recipient (e.g., a storage bag or container), so the air detector M3 may transmit signals to the controller 18 that are indicative of the presence or absence of air in the tubing. If the signal is indicative of air being present in the tubing, the controller 18 may initiate an alarm or error condition to alert an operator to the condition and/or to take corrective action to prevent the air from reaching the recipient (e.g., by reversing the flow of fluid through the tubing or diverting flow to a vent location).

The generally vertical portion 24 of the case 20 may include a plurality of volume measurement systems W1-W6 (six are shown, but more or fewer may be provided), each configured to be associated with one or more fluid containers F1-F7 of the fluid flow circuit 12 (FIG. 2). Each volume measurement system W1-W6 is configured to work in combination with the controller 18 to measure a current volume of fluid within an associated fluid container F1-F7 and to calculate a change in that volume between two or more points in time. The individual volume measurement systems W1-W6 may be variously configured without departing from the scope of the present disclosure, which may include two or more of the volume measurement systems W1-W6 being differently configured. In one exemplary embodiment, a volume measurement system W1-W6 may be configured as or include a weight scale configured to support and measure the weight of a fluid within an associated fluid container F1-F7 (with the measured weight being converted to a volume by a component of the volume measurement system W1-W6 or by the controller 18). In another exemplary embodiment, a volume measurement system W1-W6 may include one or more sensors configured to detect a volume and/or a change in volume of a fluid within an associated fluid container F1-F7. Volume measurement systems including additional components (e.g., both a weight scale and a sensor) and/or alternative components may also be employed without departing from the scope of the present disclosure.

Regardless of its particular configuration, each volume measurement system W1-W6 transmits to the controller 18 a signal that is indicative of the volume of the fluid within the associated container F1-F7 to track the change of volume during the course of a procedure. This allows the controller 18 to process the incremental volume changes to derive fluid processing volumes and flow rates and subsequently generate signals to control processing events based, at least in part, upon the derived processing volumes. For example, the controller 18 may diagnose leaks and obstructions in the fluid flow circuit 12 and alert an operator.

The illustrated case 20 is also provided with a plurality of hooks or supports H1 and H2 that may support various components of the fluid flow circuit 12 or other suitably sized and configured objects.

### D. Controller

According to an aspect of the present disclosure, the biological fluid processing device 10 includes a controller 18, which is suitably configured and/or programmed to control operation of the biological fluid processing device 10. In one embodiment, the controller 18 comprises a main processing unit (MPU), which can comprise, e.g., a Pentium^{™} type microprocessor made by Intel Corporation, although other types of conventional microprocessors can be used. In one embodiment, the controller 18 may be mounted inside the generally vertical portion 24 of the case 20, adjacent to or incorporated into an operator interface station (e.g., a touchscreen). In other embodiments, the controller 18 and operator interface station may be associated with the generally horizontal portion 22 or may be incorporated into a separate device that is connected (either physically, by a cable or the like, or wirelessly) to the biological fluid processing device 10.

The controller 18 is configured and/or programmed to execute at least one biological fluid processing procedure but, more advantageously, is configured and/or programmed to execute a variety of different biological fluid processing procedures. For example, the controller 18 may be configured and/or programmed to carry out one or more of the following: a double unit red blood cell collection procedure, a plasma collection procedure, a plasma/red blood cell collection procedure, a red blood cell/platelet/plasma collection procedure, a platelet collection procedure, and a platelet/plasma collection procedure.

More particularly, in carrying out these fluid processing procedures, the controller 18 is configured and/or programmed to control one or more of the following tasks: drawing fluid into a fluid flow circuit 12 mounted to the biological fluid processing device 10, conveying fluid through the fluid flow circuit 12 to a location for separation (i.e., into a spinning membrane separator 26 or centrifugal separation chamber 36 of the fluid flow circuit 12), separating the fluid into two or more components as desired, and conveying the separated components into storage containers, to a second location for further separation (e.g., into whichever of the spinning membrane separator 26 and centrifugal separation chamber 36 that was not used in the initial separation stage), or to a recipient (which may be a source from which the fluid was originally drawn).

This may include instructing the spinning membrane separator drive unit 14 and/or the centrifugal separator 16 to operate at a particular rotational speed and instructing a pump P1-P6 to convey fluid through a portion of the fluid flow circuit 12 at a particular flow rate. Hence, while it may be described herein that a particular component of the biological fluid processing device 10 (e.g., the spinning membrane separator drive unit 14 or the centrifugal separator 16) performs a particular function, it should be understood that that component is being controlled by the controller 18 to perform that function.

Before, during, and after a procedure, the controller 18 may receive signals from various components of the biological fluid processing device 10 (e.g., the pressure sensors A1-A4) to monitor various aspects of the operation of the biological fluid processing device 10 and characteristics of the fluid and separated fluid components as they flow through the fluid flow circuit 12. If the operation of any of the components and/or one or more characteristics of the fluid or separated fluid components is outside of an acceptable range, then the controller 18 may initiate an alarm or error condition to alert the operator and/or take action to attempt to correct the condition. The appropriate corrective action will depend upon the particular error condition and may include action that is carried out with or without the involvement of an operator.

For example, the controller 18 may include an interface control module, which receives signals from the light detector 52 of the interface monitoring system. The signals that the controller 18 receives from the light detector 52 are indicative of the location of an interface between the separated fluid components within the centrifugal separation chamber 36. If the controller 18 determines that the interface is in the wrong location, then it can issue commands to the appropriate components of the biological fluid processing device 10 to modify their operation so as to move the interface to the proper location. For example, the controller 18 may instruct one of the pumps P1-P6 to cause fluid to flow into the centrifugal separation chamber 36 at a different rate and/or for a separated fluid component to be removed from the centrifugal separation chamber 36 at a different rate and/or for the centrifugal separation chamber 36 to be spun at a different speed by the centrifugal separator 16.

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

### II. The Disposable Fluid Flow Circuit

As for the fluid flow circuit or flow set 12 (FIG. 2), it is intended to be a sterile, single use, disposable item. Before beginning a given fluid processing procedure, the operator loads various components of the fluid flow circuit 12 onto the case 20 in association with the biological fluid processing device 10. The controller 18 implements the procedure based upon preset protocols, taking into account other input from the operator. Upon completing the procedure, the operator removes the fluid flow circuit 12 from association with the biological fluid processing device 10. The portions of the fluid flow circuit 12 holding the collected fluid component or components (e.g., collection containers or bags) are removed from the case 20 and retained for storage, transfusion, or further processing. The remainder of the fluid flow circuit 12 is removed from the case 20 and discarded.

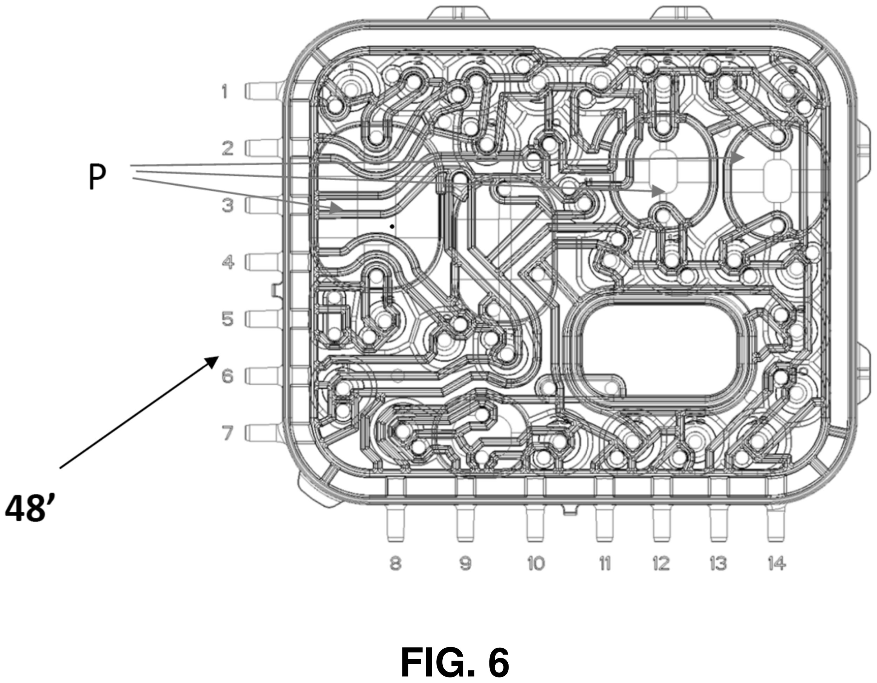

In the illustrated embodiment, the fluid flow circuit 12 includes a cassette 48 of the type shown in Fig. 5 or 48' shown in Fig. 6, to which the other components of the fluid flow circuit 12 are connected by flexible tubing defining flow paths. The other components may include a plurality of fluid containers F1-F7. In the context of the present disclosure these containers include an anticoagulant container F1, a saline container F2, an in-process container F3, a return container F4, a plasma collection container F5, a platelet collection container F6, and an (optional) additive container F7. The illustrated flow circuit 12 further includes one or more fluid source access devices (e.g., a connector or spike for accessing blood within a fluid container or a phlebotomy needle), a spinning membrane separator 26 and a centrifugal separation chamber 36.

The flow control cassette 48 (Fig. 5) or 48' (Fig. 6) provides a centralized, programmable, integrated platform for all the pumping and many of the valving, sensing or other functions required for a given fluid processing procedure. In one embodiment, the cassette is similarly configured to the cassette of U.S. Patent No. 5,868,696, but is adapted to include additional components (e.g., more tubing loops) and functionality.

In use, the cassette 48 is mounted to the cassette station 54 of the biological fluid processing device 10 so as to align one or more of sensor stations S1-S4 with an associated pressure sensor A1-A4 of the cassette station 54 and its valve stations with an associated valve V1-V9. Each valve station may define one or more ports that allow fluid communication between the valve station and another interior cavity of the cassette (e.g., a flow path). As described above, each valve V1-V9 is movable under command of the controller 18 to move between a plurality of positions (e.g., between a retracted or lowered position and an actuated or raised position) to selectively contact the valve stations of the cassette. In the actuated position, a valve V1-V9 engages the associated valve station to close one or more of its ports to prevent fluid flow therethrough. In the retracted position, a valve V1-V9 is disengaged from the associated valve station (or less forcefully contacts the associated valve station than when in the actuated position) to open one or more ports associated with the valve station, thereby allowing fluid flow therethrough.

A plurality of tubing loops extend from the side surface of the cassette to interact with pumps P1-P6 of the biological fluid processing device 10. The different pumps P1-P6 may interact with the tubing loops of the cassette to perform different tasks during a procedure, but in the context of the present disclosure, a different one of the pumps P1-P6 may be configured to serve as an anticoagulant pump P1, a source pump P2, a centrifuge pump P3, an outlet pump P4, a recirculation pump P5, and a plasma pump P6. If the pumps P1-P6 are differently configured (e.g., if they are configured as pneumatic pumps as shown in Fig. 6)), then the cassette may be differently configured (e.g., with pump stations aligned with pneumatic pump actuators) to allow for the pumps P1-P6 to convey fluid through the cassette.

Additional tubing extends from the side surface of the cassette to connect to the other components of the fluid flow circuit 12, such as the various fluid containers F1-F7, the spinning membrane separator 26, and the centrifugal separation chamber 36. The tubing connected to the centrifugal separator chamber 36 (which includes one inlet tube and two outlet tubes) may be aggregated into an umbilicus.

Various additional components may be incorporated into the tubing leading out of the cassette or into one of the cavities of the cassette. For example, a manual clamp 56 may be associated with a line or lines leading to the fluid source, a return line filter 58 (e.g., a microaggregate filter) may be associated with a line leading to a fluid recipient, and/or an air trap 62 may be positioned on a line upstream of the centrifugal separation chamber 36.

### III. Anomaly Detection with Pressure Sensors

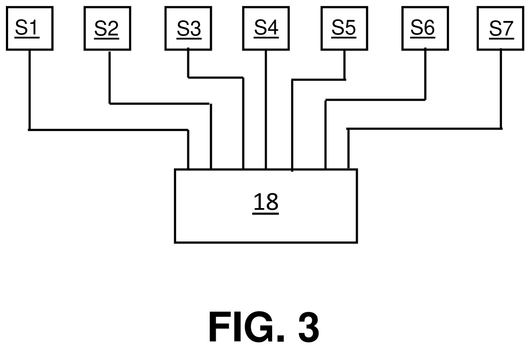

The processing device 10, via the controller 18, may be configured to detect flow conditions considered to be anomalous with respect to the various containers F1-F7 and tubing connected directly below the containers F1-F7. In the present embodiments, a container pressure sensor system S1-S7 includes one or more container pressure sensors S1-S7 sense pressure in tubing directly below a container. The controller 18 may detect the condition(s) using signals received from one or more container pressure sensors S1-S7 to measure the pressure exerted by the volume of a fluid in the corresponding container F1-F7. The present disclosure is not limited to the number and position of container pressure sensors S1-S7 in the illustrated examples of FIGS. 2 and 3. For example, container pressure sensors S1-S7 may be provided to detect pressure in the tubing connected and directly below, some, but not all, of the containers F1-F7. In some examples, a single container pressure sensor S1-S7 may be connected to a tube downstream from multiple containers F1-F7.

With further reference to FIGS. 2 and 3, the processing device 10 includes the one or more container pressure sensors S1-S7 which are communicably connected to the controller 18 to transmit signals to controller 18 indicative of the pressure in the tube directly below a container F1-F7. Thus, before, during and/or after a procedure, the controller 18 may receive the signals from the container pressure sensors S1-S7 to monitor the pressure in the tubes directly below the containers F1-F7, which is the pressure exerted by the volume of a fluid in the container F1-F7. The controller 18 may then determine one or more conditions based on the received signals. Such conditions include, for example, emptying of the containers F1-F7, filling of the containers F1-F7 and/or air in the tube directly below the containers F1-F7. In some examples, the controller 18 may also determine a volume of fluid in the containers F1-F7 based on the signals received from the container pressure sensors S1-S7.

The conditions detected using the signals from the container pressure sensors S1-S7 may be considered anomalous in some instances because continued operation of the processing device 10 in a manner which would further the conditions should generally be avoided. In some instances, the conditions may be indicative of an anomalous event occurring during the procedure or the processing device 10 not operating in an intended manner. For example, such conditions may arise when a component of the processing device 10, such as the pumps P1-P6 or valves V1-V9 do not operate as intended.

The controller 18 may control the processing device 10 in response to detecting one of more the conditions determined using the signals received from the container pressure sensors S1-S7. For example, the controller 18 may control one or more components of the processing device 10, such as the centrifuge 16, pumps P1-P6 and/or valves V1-V9 to prevent furtherance of the detected condition, and in some embodiments, to correct the detected condition and continue the procedure. Alternatively, or additionally, the controller 18 may also operate the processing device 10 to output a message to an operator or user, output an alert, halt the procedure, and/or provide an instruction to an operator describing action needed before resuming procedures.

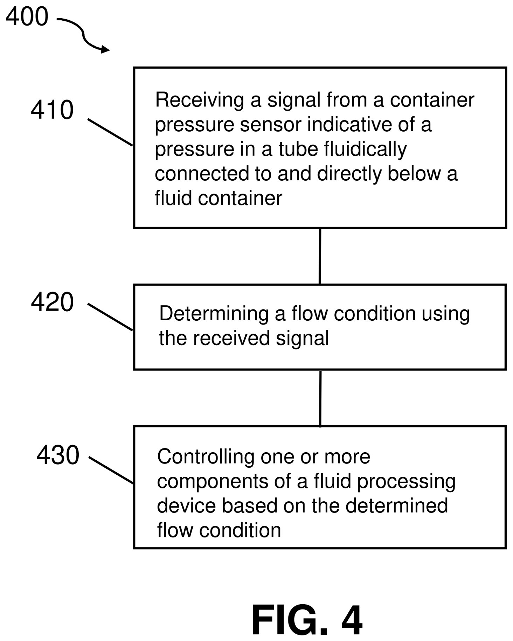

Referring to FIG. 4, a method 400 of detecting a flow condition, and in particular an anomalous condition, in fluid processing procedure may include receiving 410 a signal from the container pressure sensor system S1-S7 at the controller 18. The received signal is indicative of a pressure in a flow path (such as a tube, or cassette flow path) fluidically connected to and positioned directly below a fluid container F1-F7. In various embodiments, the signal may be received continuously by the controller 18, at predetermined time intervals, or in response to other detected events. Alternatively, or in addition, in some examples the controller 18 may receive the signals continuously and may sample the received signal continuously to monitor pressure in the flow path directly below an associated container F1-F7. Alternatively, or in addition, the controller 18 may sample the received signal at predetermined time intervals or in response to other detected events. At 420, the method may include determining the condition using the received signals from the container pressure sensors S1-S7. The determined condition may be a first condition indicative of fluid container emptying, a second condition indicative of fluid container filling and a third condition indicative of air in the tube at a container pressure sensor. Inasmuch as the system tracks the volumes of liquid being delivered by, for example, pump strokes completed (pneumatic) or revolutions (peristaltic) or by a weight scale reading (i.e. increase in weight over an original tare), monitoring the performance of pumps P1-P6, or valves V1-V9 and/or the readings of the pressure sensors also serves as a redundant safety monitor for the system.

In some examples, the determining of the condition may use a series of received signals and the controller 18 may further process the signals to determine the flow condition. In various examples which are described further below, the controller 18 may process the received signals to determine or measure the pressure, a change in pressure, and/or a rate of change of the pressure, in the flow path (tube) directly below the container F1-F7. In some examples, the received signals, or information derived from the signals (e.g., pressure, change in pressure, rate of change of pressure, etc.) may be compared to a stored baseline or threshold value (or range). The baseline or threshold value may be determined by the controller 18 using the received signals when a known condition is occurring, i.e., with an empty container, a filling container, air passing through tube at the container pressure sensor S1-S7, etc. The determined or measured value(s) may then be associated with a corresponding condition and stored in the memory.

It is also envisioned that the controller 18 may determine a standard deviation of the received signals, or information determined based on the received signals, and determine the condition based on the standard deviation, a change in the standard deviation, comparison to a threshold standard deviation value, and the like.

The method may also include, at 430, controlling the processing device 10 based on the detected condition. For example, the controller 18 may be configured to control one or more components of the processing device 10, such as the centrifuge 16, one or more pumps P1-P6, one or more valves V1-V9, a communication interface facilitating wired and/or wireless communications to transmit information about the detected condition, and/or an operator interface to provide information to the user about the detected condition. In one example, the controller 18 may control the pump system P1-P6 to stop drawing fluid from a fluid container if the controller 18 determines the fluid container is emptied to limit or prevent air from the empty fluid container being drawn into the tubes. The controller 18 could also control the pump system P1-P6 to begin filling fluid container that has been detected as emptied. The controller may also be configured/programmed to prevent the emptying from occurring again in the future, such as re-taring the scale (correcting the volume in the container as read by the controller) or adjusting the stroke volume calculation in the controller for pumps P1-P6 to better account for the volume which is actually being moved by the pump.

The operator interface may be a graphical interface such as a display, one or more lights indicative of a condition being detected and/or for alerting the user that intervention may be needed, a speaker for outputting an alarm or audio message, and other known, suitable user interfaces.

It will also be appreciated that the controller 18 may control one or more components of the processing device 10 by activating or deactivating the component, changing an operating parameter of the component (e.g., speed, power, position, etc.), and/or maintaining an operating parameter of a component at a current level.

Thus, in present examples, the processing device 10 may include the container pressure sensor system having one or more container pressure sensors S1-S7 for sensing and monitoring pressure in tubing directly below a container. The one or more container pressure sensors S1-S7 may then be used to measure the pressure exerted by the volume of a fluid in the corresponding container, which may also be referred to as the "head-height" for the container. The processing device 10, via the controller 18, may then determine whether an event or condition has occurred or is occurring at the container, such as emptying or filling, using the measured pressure.

With reference to FIGS. 2 and 3, in the present examples a container pressure sensor S1-S7 may be arranged in the tubing directly below a container F1-F7. In some examples, a plurality of container pressure sensors S1-S7 may be provided for respective containers F1-F7. The container pressure sensor S1-S7 is operably connected to the controller 18 and is configured to transmit a signal to the controller 18 indicative of the pressure detected by the sensor S1-S7. The controller 18 may process the signal received from the container pressure sensor S1-S7 to determine the pressure in the corresponding tube below the container F1-F7. The controller 18 may store the measured pressures in a memory associated with the controller 18. In some examples, the measured pressures may be stored with corresponding time stamps and/or identification information for the container pressure sensor from which the signal was received to determine the measured pressure, the corresponding container, and/or the fluid in the container (if any) when the measured was determined.

In one example, the controller 18 may be configured to compare the measured pressure to the baseline pressure. The controller 18 may then determine an approximate volume of fluid in the container based on the comparison between the measured pressure and the baseline pressure and the specific gravity of the fluid, which may be stored in the memory.

If the measured pressure is equal to or substantially equal to the baseline pressure, the controller 18 may determine that the container F1-F7 is empty and control the processing device 10 to take steps to prevent over-emptying of the container F1-F7. That is, the controller 18 may control the centrifuge 16 and/or the various pumps P1-P6 and/or valves V1-V9 as needed to prevent over-emptying. In one example, the controller 18 may control the processing device 10 to perform a state transition, i.e., to begin filling the container F1-F7 after detecting an emptying condition or stopping the pumps P1-P6 that are emptying the container F1-F7 to limit or prevent overemptying. In addition, or alternatively, the controller 18 may generate an alert to be output by the processing device 10, the alert indicating, for example, that a state transition is going to occur or is occurring, that user attention or intervention may be needed, and/or that the procedure has been stopped.

Conversely, if the measured pressure has increased from the baseline pressure, the controller 18 may determine that the container F1-F7 is filling. For example, in a priming stage, during which air may be removed from tubes, the measured pressure will increase from the baseline pressure as a container F1-F7 or corresponding tubing is filled with fluid, such as saline or blood. If the controller determines the container F1-F7 to be full, for example by comparing the measured pressure to a maximum threshold pressure corresponding to a full container, the controller 18 may control processing device 10 to prevent further filling of the container F1-F7, for example, by operating one or more of the centrifuge 16, pumps P1-P6 and/or valves V1-V9 to prevent fluid from flowing into the container F1-F7.

In some configurations, there may be multiple containers F1-F7 connected on a same tubing line. A pressure detected by a container pressure sensor S1-S7 on such a tubing line may not be representative of the volume of fluid in a single container. In addition, the container pressure sensor S1-S7 may not be able to provide a redundant safety monitor (as described above) to confirm the procedure driver/controller is responding accordingly.

Thus, in one example, a preset threshold pressure may be set and the controller 18 may compare a measured pressure to the preset threshold pressure. If the measured pressure exceeds the preset threshold pressure, then the controller 18 may control the processing device 10 to take steps to prevent air introduction. Conversely, the controller 18 may control the processing device 10 to continue operating according to current parameters if the measured pressure is less than the preset threshold pressure.

Controller 18 may monitor a negative pressure on the container F1-F7 and then further monitor an increase from the negative pressure. The controller 18 may perform a gross monitor check function in a similar manner, to confirm pump operation and/or pump roller integrity. For example, if one or more pumps are not operating as intended, the measured pressure would not be able to be built or released. A pump may not operate as intended, for example, if a roller on a peristaltic pump is broken or not occluding or one or more valves on a pneumatic pump were non-functional or not occluding.

Alternatively, or in addition, in some examples, the pressure may be periodically measured by the controller 18, based on periodic sampling of the container pressure sensor S1-S9, and the measured pressure may be stored as a function of time. In general, the pressure in the tube when fluid is present in a container is relatively stable regardless of the volume in the container F1-F7. However, the pressure drops rapidly when a container F1-F7 empties, and air is drawn into the tube.

In one example, the controller 18 may periodically sample the pressure sensor reading and calculate the standard deviation of the pressure. The controller 18 may then compare the standard deviation to a threshold and determine when the container F1-F7 transitions from having fluid inside to being emptied based on the standard deviation. For example, an increase in the standard deviation would be indicative of pressure instability. Thus, if the controller 18 determines the standard deviation to be increasing, the controller 18 may determine that the container F1-F7 has transitioned to empty. The controller 18 can control the processing device 10 to respond accordingly to prevent air from being introduced into the rest of the disposable set in response to determining the container F1-F7 is emptied.

Conversely, the pressure in the tube may be stable when fluid is not present. The pressure rises rapidly when fluid is introduced to fill the container F1-F7. Accordingly, the controller 18 may compare the standard deviation to a separate standard deviation threshold and may determine that the container F1-F7 begins filling based on the comparison.

As described above, in some examples, the controller 18 is configured to periodically sample the detected pressure by the container pressure sensor to determine measure pressures as a function of time. In one example, the controller 18 may compare the currently measured pressure to a previously measured pressure, determine a slope of the pressure curve. The controller 18 may then compare the determined slope to a threshold slope. The controller 18 may determine the container F1-F7 is emptied, for example, if the determined slope is negative, indicating a drop in pressure, and has a magnitude exceeding a threshold negative slope.

Conversely, the controller 18 may determine the container F1-F7 is filling if the determined slope is positive and exceeds the threshold positive slope. The controller 18 may control the processing device 10 depending on the determined condition. The larger the slope of the line created by one or more measured pressures, or the same slope turning negative in general, can be used as a trigger for the system to respond, for example, by controlling one or more components of the processing device 10 as described above.

In still other examples, the controller 18 may be configured to determine the presence or absence of air in the tube based on the signal received from the container pressure sensor S1-S7, and thus, may be used as a local air detector. Such detection may be accomplished without the need for hardware and electronics associated with more precise air detectors (e.g., ultrasonic air detectors). For example, the controller 18 may continuously sample and monitor the signal received from the container pressure sensor S1-S7 and determine changes in the pressure with respect to time. In one example, when air passes over the sensor S1-S7, a transitory dip in the measured pressure will occur. Once the air has passed and the liquid flow by the container pressure sensor S1-S7 resumes, the measured pressure will quickly increase, for example, to a value at or near the pressure before the air passed over the sensor S1-S7. The length of the detected pressure dip generally corresponds to the amount of air passing over the sensor S1-S7, with both amplitude and length of time of the pressure dip. Detecting the presence of air may be useful, for example, to detect if a needle has fallen out of donor's arm, which would provide a larger signal response (i.e., long time duration, higher amplitude) or as a secondary or tertiary check if an air purge has likely been successful, which would provide a small signal response.

The methods above can be performed continuously throughout a blood processing procedure or at specific times. In addition, the above methods may also incorporate information received from other sensors or inputs in the system. For example, the controller 18 may also reference information received from one or more flow sensors which measure fluid flow at or near one or more of the pumps P1-P6. In one example, if the controller 18 detects a condition or status transition, i.e., emptying or filling, or air passing the sensor S1-S7, a baseline of the associated pump volumes can be taken based on flow sensor information and/or pump operating information to further increase accuracy of the future event detections. That is, the controller 18 may calculate a volume of fluid expected to have been pumped into a container F1-F7 based on the flow sensor information or pump operating information. The controller 18 may also calculate a volume of fluid expected to have been drawn from the container F1-F7 based on similar information. During a blood processing procedure, the controller 18 may compare the calculated fluid volume into the container F1-F7 and the calculated fluid volume out of the container F1-F7. If the controller 18 determines that the calculated fluid volume in and fluid volume out values are approaching one another, or are substantially equal, the controller 18 may then activate a pressure monitoring function using the container pressure sensors S1-S7. In this manner, detection of a condition or status transition, e.g., emptying or filling, may generally be limited to time periods when such transitions are believed to be most likely. By limiting the monitoring of the container pressure sensors S1-S7, false positives may be reduced or avoided.

In some examples, the controller 18 may use the container pressure sensor signals and measured pressures to confirm or improve accuracy of other sensor measurements. In turn, the determination of a container status, i.e., filling or emptying, may be used to confirm or improve accuracy of other sensor measurements. For example, the controller 18 may use weight information from the weight sensors and/or flow information from flow sensors to determine a volume of fluid in the container F1-F7. The controller 18 may compare the calculated volume values to fluid volumes in the containers F1-F7 determined using the container pressure sensors S1-S7. In the event a difference between the calculated volumes using different sensor information exceeds a threshold, the controller 18 may control the processing device 10 to adjust parameters of the various components as needed to reduce the difference in values.

In some examples, the controller 18 may be configured to provide an initial "blackout" period when the pumps P1-P6 begin to draw fluid from a container F1-F7. In The controller 18 does not use measured pressures which would have been obtained during the blackout period. For example, a detected pressure often decreases when the pumps are operated for an initial withdrawal of fluid from a container F1-F7. Such a decrease in pressure may resemble a decrease in pressure that occurs when the container F1-F7 is emptied. Thus, by implementing the blackout period, the controller 18 may avoid processing sensor signals which may result in a false positive indication of container emptying.

The controller 18 may determine the blackout period based on, for example, the amount of time that has elapsed since the pump(s) P1-P6 started and/or the volume pumped. Alternatively, or in addition, the blackout period could be dynamically adjusted based on the rate of the pump(s) P1-P6 with slower overall speeds using a shorter blackout period and faster rates using a longer blackout period. It may be beneficial to minimize the blackout period to reduce likelihood of blacking out pressure signal indicative of an actual emptying event. After the blackout period, the measured pressure will settle around a relatively stable pressure value.

In some examples, the controller 18 may monitor the length of time and/or the volume of fluid moved by the pump(s) P1-P6 after determining a condition or status, i.e., empty, filling, air detected. The controller 18 may compare the monitored length of time or volume of fluid to an amount of time and/or volume of fluid it takes for the container pressure sensor S1-S7 to begin indicating that there is the opposite status, e.g., filling to empty, empty to filling, air detected again, after reversing pump direction to confirm that the status detection was accurate and likely not a false positive. If the amount of time and/or volume of fluid monitored closely match the amount of time and/or volume of fluid the controller estimates to have elapsed, additional steps may need to be performed to prevent future events from occurring, such as adjusting configurations/stroke volumes etc. or notifying the operator to confirm a condition (i.e. that the container is/is not empty or assist in rectifying the condition (add a new Saline container for example)).On the other hand, if the amount of time and/or volume of fluid monitored do not closely match, future monitoring for the condition may be required (i.e. at the next expected point where the container may empty/filling etc. or for future indications that the container is empty/filling etc. Such steps may be useful to reduce the likelihood the controller 18 does not take unnecessary steps to control the processing the device 10 to correct behavior which was anomalous.

### V. Aspects

Aspect 1. A fluid processing device includes one or more components configured for controlling flow through a flow circuit, the one or more components including one or more of a centrifuge, a pump system and a valve system; a container pressure sensor system including one or more container pressure sensors configured to be associated to a tube fluidically connected to a fluid container and positioned directly below the container. The container pressure sensor system is configured to detect and transmit signals indicative of a pressure within the tube, wherein the pressure corresponds to a pressure exerted by a volume of fluid in the associated container. The fluid processing device also includes a controller operably connected to the container pressure sensor system and configured to receive the signals transmitted from the container pressure sensor system, determine a flow condition using the received signals, and control the one or more components based on the determined flow condition.

Aspect 2. The fluid processing device of Aspect 1, wherein the controller is configured to continuously sample signals received from the container pressure sensor system.

Aspect 3. The fluid processing device of Aspect 1, wherein the controller is configured to sample signals received from the container pressure sensor system at predetermined intervals.

Aspect 4. The fluid processing device of Aspect 1, wherein the controller is configured to sample signals received from the container pressure sensor system in response to a detected event.

Aspect 5. The fluid processing device of any of Aspects 1-4, wherein the controller is configured to determine one or more of a pressure reading, a change in the pressure, and a rate of change of the pressure using the signals received from the container pressure sensor system, and determine the flow condition based on one or more of the pressure, change in pressure and rate of change of the pressure.

Aspect 6. The fluid processing device of any of Aspects 1-5, wherein the controller is configured to determine a standard deviation of the signals received from the container pressure sensor system and determine the flow condition based on the standard deviation.

Aspect 7. The fluid processing device of any of Aspects 1-6, wherein the controller is configured to control the pump system based on the determined flow condition.

Aspect 8. The fluid processing device of any of Aspects 1-7, wherein the controller is configured to generate an alert to a user based on the determined flow condition.

Aspect 9. The fluid processing device of any of Aspects 1-8, wherein the determined flow condition is one or more of a first condition indicative of the fluid container emptying, a second condition indicative of the fluid container filling, and a third condition indicative of air in the tube at a container pressure sensor of the container pressure sensor system.

Aspect 10. A fluid processing system includes a fluid processing device and a disposable fluid flow circuit configured for selective arrangement on the fluid processing device for the fluid processing device to perform a procedure. The fluid processing device includes one or more components including a pump system, a valve system, and a centrifuge, a container pressure sensor system including one or more container pressure sensors, and a controller operably connected to the container pressure sensor system. The disposable fluid flow circuit includes one or more fluid containers and one or more tubes fluidically connected to the one or more fluid containers to allow fluid flow into and out of the one or more containers. The disposable fluid flow circuit is arranged on the fluid processing device such that a container pressure sensor of the container pressure sensor system is arranged at a tube of the one or more tubes directly below at least one fluid container of the one or more fluid containers to which to the tube is fluidically connected. The container pressure sensor system is configured to detect and transmit signals indicative of a pressure within the tube, wherein the pressure corresponds to a pressure exerted by a volume of fluid in the corresponding fluid container. The controller is configured to receive the signals transmitted from the container pressure sensor system, determine a flow condition using the received signals, and control the one or more components based on the determined flow condition.

Aspect 11. The fluid processing system of Aspect 9, wherein the determined flow condition is one or more of a first condition indicative of the fluid container emptying, a second condition indicative of the fluid container filling, and a third condition indicative of air in the tube at the pressure sensor.

Aspect 12. A method of determining a flow condition in a fluid processing procedure is performed by a fluid processing device. The fluid processing device includes one or more components configured to direct fluid flow, a container pressure sensor system comprising one or more pressure sensors, and a controller operably connected to the container pressure sensor system and at least one of the one or more components. The method includes receiving a signal from a container pressure sensor of the container pressure sensor system indicative of a pressure in a tube fluidically connected to and directly below a fluid container, determining a flow condition using the received signal, and controlling the at least one component of the one or more components based on the determined flow condition.

Aspect 13. The method of Aspect 12, wherein receiving the signal includes continuously sample signals received from a container pressure sensor.

Aspect 14. The method of Aspect 12, wherein receiving the signal includes sampling signals received from the container pressure sensor at predetermined intervals.

Aspect 15. The method of Aspect 12, wherein receiving the signal includes sampling signals received from the container pressure sensor in response to a detected event.

Aspect 16. The method of any of Aspects 12-15, wherein determining the flow condition includes determining one or more of a pressure readings, a change in the pressure, and a rate of change of the pressure using the signals received from the container pressure sensor system, wherein the flow condition is determined based on one or more of the pressure, change in pressure and rate of change of the pressure.

Aspect 17. The method of any of Aspects 12-16, wherein determining the flow condition includes determining a standard deviation of the signals received from the container pressure sensor system, wherein the flow condition is determined based on the standard deviation.

Aspect 18. The method of any of Aspects 12-17, wherein controlling the at least one component includes controlling the pump system based on the determined flow condition.

Aspect 19. The method of any of Aspects 12-18, wherein controlling the at least one component includes outputting an alert to a user based on the determined flow condition.

Aspect 20. The method of any of Aspects 12-19, wherein the determined flow condition is one or more of a first condition indicative of the fluid container emptying, a second condition indicative of the fluid container filling, and a third condition indicative of air in the tube at a container pressure sensor of the container pressure sensor system.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A fluid processing device comprising:
one or more components configured for controlling flow through a flow circuit, the one or more components including one or more of a centrifuge, a pump system and a valve system;
a container pressure sensor system comprising one or more container pressure sensors configured to be associated to a tube fluidically connected to a fluid container and positioned directly below the container, the container pressure sensor system configured to detect and transmit signals indicative of a pressure within the tube, wherein the pressure corresponds to a pressure exerted by a volume of fluid in the associated container; and
a controller operably connected to the container pressure sensor system and configured to:
receive the signals transmitted from the container pressure sensor system;
determine a flow condition using the received signals; and
control the one or more components based on the determined flow condition.

2. The fluid processing device of claim 1, wherein the controller is configured
- to continuously sample signals received from the container pressure sensor system, and/or
- to sample signals received from the container pressure sensor system at predetermined intervals, and/or
- to sample signals received from the container pressure sensor system in response to a detected event.

3. The fluid processing device of any of claims 1-2, wherein the controller is configured to determine one or more of a pressure, a change in the pressure, and a rate of change of the pressure using the signals received from the container pressure sensor system, and determine the flow condition based on one or more of the pressure, change in pressure and rate of change of the pressure.

4. The fluid processing device of any of claims 1-3, wherein the controller is configured
- to determine a standard deviation of the signals received from the container pressure sensor system and determine the flow condition based on the standard deviation, and/or
- to control the pump system based on the determined flow condition, and/or
- to generate an alert to a user based on the determined flow condition.

5. The fluid processing device of any of claims 1-4, wherein the determined flow condition is one or more of a first condition indicative of the fluid container emptying, a second condition indicative of the fluid container filling, and a third condition indicative of air in the tube at a container pressure sensor of the container pressure sensor system.

6. A fluid processing system comprising:
a fluid processing device and a disposable fluid flow circuit configured for selective arrangement on the fluid processing device for the fluid processing device to perform a procedure,
wherein the fluid processing device comprises:
one or more components, the one or more components including a pump system, a valve system, and a centrifuge;
a container pressure sensor system including one or more container pressure sensors; and
a controller operably connected to the container pressure sensor system,
wherein the disposable fluid flow circuit comprises:
one or more fluid containers; and
one or more tubes fluidically connected to the one or more fluid containers to allow fluid flow into and out of the one or more containers,
wherein the disposable fluid flow circuit is arranged on the fluid processing device such that a container pressure sensor of the container pressure system is arranged at a tube of the one or more tubes directly below at least one fluid container of the one or more fluid containers to which to the tube is fluidically connected,
wherein the container pressure sensor system is configured to detect and transmit signals indicative of a pressure within the tube, wherein the pressure corresponds to a pressure exerted by a volume of fluid in the corresponding fluid container, and
wherein the controller is configured to:
receive the signals transmitted from the container pressure sensor system,
determine a flow condition using the received signals; and
control the one or more components based on the determined flow condition.

7. The fluid processing system of claim 6, wherein the determined flow condition is one or more of a first condition indicative of the fluid container emptying, a second condition indicative of the fluid container filling, and a third condition indicative of air in the tube at the pressure sensor.

8. A method of determining a flow condition in a fluid processing procedure, wherein the fluid processing procedure is performed by a fluid processing device comprising one or more components configured to direct fluid flow, a container pressure sensor system comprising one or more pressure sensors, and a controller operably connected to the container pressure sensor system and at least one of the one or more components, the method comprising:
receiving a signal from a container pressure sensor of the container pressure sensor system indicative of a pressure in a tube fluidically connected to and directly below a fluid container;
determining a flow condition using the received signal; and
controlling the at least one component of the one or more components based on the determined flow condition.

9. The method of claim 8, wherein receiving the signal includes
- continuously sample signals received from a container pressure sensor, and/or
- sampling signals received from the container pressure sensor at predetermined intervals, and/ or
- sampling signals received from the container pressure sensor in response to a detected event.

10. The method of any of claims 8-9, wherein determining the flow condition includes determining one or more of a pressure, a change in the pressure, and a rate of change of the pressure using the signals received from the container pressure sensor system, wherein the flow condition is determined based on one or more of the pressure, change in pressure and rate of change of the pressure, and/or determining a standard deviation of the signals received from the container pressure sensor system, wherein the flow condition is determined based on the standard deviation.

11. The method of any of claims 8-10, wherein controlling the at least one component includes controlling the pump system based on the determined flow condition, and/or outputting an alert to a user based on the determined flow condition.

12. The method of any of claims 8-11, wherein the determined flow condition is one or more of a first condition indicative of the fluid container emptying, a second condition indicative of the fluid container filling, and a third condition indicative of air in the tube at a container pressure sensor of the container pressure sensor system.

13. The method of any one of claims 8-12 comprising calculating a volume of fluid expected to have been pumped into a container based on flow sensor information or pump operating information and/or calculating a volume of fluid expected to have been drawn from the container based on . flow sensor information or pump operating information.

14. The method of any one of claims 8-13 comprising providing a blackout period during which measured pressures which would have been obtained are not monitored.

15. The method of Claim 8 wherein the controller is configured to monitor the length of time and/or the volume of fluid moved by the pump(s) after determining a condition or status of a container, in particular wherein the controller is configured to compare the monitored length of time or volume of fluid to an amount of time and/or volume of fluid it takes for the container pressure sensor to begin indicating that there is the opposite status.
